Europäisches Patentamt

European Patent Office

Office européen des brevets

(19)

(11) Veröffentlichungsnummer : **0 197 447**
**B1**

(12) **EUROPÄISCHE PATENTSCHRIFT**

(45) Veröffentlichungstag der Patentschrift :
30.08.89

(21) Anmeldenummer : 86104215.8

(22) Anmeldetag : 26.03.86

(51) Int. Cl.⁴ : **C 12 N 9/96**, G 01 N 33/535,
G 01 N 33/74, G 01 N 33/78,
G 01 N 33/68, G 01 N 33/574

(54) Stabilisierung der Aktivität von Peroxidase in Lösung.

(30) Priorität : 28.03.85 DE 3511327

(43) Veröffentlichungstag der Anmeldung :
15.10.86 Patentblatt 86/42

(45) Bekanntmachung des Hinweises auf die Patenterteilung : 30.08.89 Patentblatt 89/35

(84) Benannte Vertragsstaaten :
AT BE CH DE FR GB IT LI LU NL SE

(56) Entgegenhaltungen :
EP–A– 0 024 578
EP–A– 0 070 992
US–A– 4 252 896
US–A– 4 504 579
Die Akte enthält technische Angaben, die nach dem Eingang der Anmeldung eingereicht wurden und die nicht in dieser Patentschrift enthalten sind.

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Klenner, Dagmar, Dr. rer. nat.
Bahnhofstrasse 54
D-8133 Feldafing (DE)
Erfinder : Kleinhammer, Gerd, Dr. rer. nat.
Kreuzeckstrasse 3
D-8132 Tutzing (DE)
Erfinder : Deeg, Rolf, Dr. rer. nat.
Hirtenstrasse 7
D-8139 Bernried (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

EP 0 197 447 B1

Jouve, 18, rue St-Denis, 75001 Paris, France

## Beschreibung

Peroxidase ist ein vielverwendetes Enzym, insbesondere im Rahmen enzymatischer Nachweisreaktionen. Auch im Rahmen der vielfältigen Ausgestaltungen von Enzymimmuntests (EIA) hat die Peroxidase als Markierungsenzym große Verbreitung gefunden. Peroxidasen verschiedener Herkunft, insbesondere die Meerettich-Peroxidase, lassen sich mit einer Vielzahl von Methoden rasch und quantitativ nachweisen.

Hierauf beruht die sehr gute Eignung der Peroxidase als Nachweis- oder Markierungsenzym. Andererseits weist die Peroxidase (POD) jedoch den Nachteil auf, daß sie speziell in höherer Verdünnung und in Lösung eine nicht ganz befriedigende Aktivitätsstabilität aufweist. Die Lebensdauer insbesondere von Peroxidase-Konjugaten im Rahmen enzymatischer Testreagenzien oder anderer Enzympräparate ist daher beschränkt und um die angestrebte Lagerungsfähigkeit zu erzielen, ist eine Stabilisierung nötig.

Aus der DE 31 00 076 Al ist es bereits bekannt, Peroxidase in einem Serumprotein enthaltenden Medium durch Zugabe von 8-Anilino-1-naphthalin-sulfonsäure (ANS) zu stabilisieren. Aus der EP 0 070 992 Al ist es bekannt, zur Stabilisierung der POD in einem Serum oder Serumprotein enthaltenden Medium 4-Aminoantipyrin zu verwenden. Aus der USA 4 169 012 A ist es schließlich bekannt, POD durch polyvalente Ionen aus den Perioden 3 und 4 des Periodensystems wie zum Beispiel Al, Zn, Mg, Fe und Cu zu stabilisieren. Diese bekannten Aktivitätsstabilisatoren vertragen sich mit anderen Zusätzen üblicher Reagenzkombinationen schlecht oder lassen hinsichtlich der Aktivitätsstabilisierung immer noch zu wünschen übrig. Außerdem beeinflussen sie teilweise die Immunreaktion negativ, wenn die POD als Konjugat mit einer immunologisch wirksamen Substanz vorliegt, was zu sehr flachen Eichkurven führt.

Der Erfindung liegt daher die Aufgabe zugrunde, die Aktivität von Peroxidase oder Peroxidase-Konjugaten in Lösung ohne die genannten Nachteile und ohne nachteilige Beeinflussung der eigentlichen Immunreaktion zu stabilisieren.

Gelöst wird diese Aufgabe erfindungsgemäß durch ein Verfahren zum Stabilisieren der Aktivität von Peroxidase in Lösung durch Zusatz eines spezifischen Aktivitätsstabilisators, welches dadurch gekennzeichnet ist, daß man dem in fester oder gelöster Form vorliegenden Enzym als Aktivitätsstabilisator Aminopyrin in einer Menge von 0,0005 bis 2 Gew.-%, bezogen auf die Lösung, zusetzt.

Erfindungsgemäß wird die Enzymaktivität der frei oder gegebenenfalls auch in einem Konjugat kovalent gebunden vorliegenden Peroxidase gegen den besonders in verdünnten Lösungen sehr ausgeprägten inaktivierenden Einfluß von Temperatur, Fremdsubstanzen und dergleichen stabilisiert. Dies bedeutet, daß die in Lösung auftretende rasche Abnahme der Enzymaktivität beseitigt bzw. stark vermindert wird, ohne daß hierdurch die immunologische Wirksamkeit einer an die Peroxidase gebundenen, immunologisch wirksamen Substanz im Konjugat beeinträchtigt wird.

Der erfindungsgemäße Aktivitätsstabilisator wird wie oben bereits erwähnt, in einer Menge zwischen 0,0005 und 2 Gew.-%, bezogen auf das Volumen der Lösung, in der die POD enthalten ist, zugesetzt. Bei Überschreitung der Obergrenze von 2 % bleibt zwar die Belastungsfähigkeit gegenüber Wärmeeinwirkung nach wie vor erhalten, die POD-Aktivität selbst wird jedoch wieder herabgesetzt. Unterhalb der unteren Grenze wird keine zufriedenstellende Stabilisierung mehr erzielt. Bevorzugt wird der Aktivitätsstabilisator gemäß der Erfindung in Mengen zwischen 0,005 und 1,0 Gew.-% zugesetzt.

Der Aktivitätsstabilisator kann dem in fester oder gelöster Form vorliegenden Enzym oder Enzymkonjugat zu einem beliebigen Zeitpunkt zugesetzt werden. Bevorzugt wird ein Zusatz zur Lösung, da hierdurch eine gleichmäßigere Verteilung des Stabilisators und damit eine bessere Wirkung auch bei Zusätzen an der unteren Grenze des wirksamen Bereiches erzielt wird. Insbesondere kann man das Aminopyrin der Enzym- bzw. Konjugatlösung vor der Lyophilisierung oder nach dem Rekonstituieren des Lyophilisats mit einem wäßrigen Lösungsmittel zusetzen, wobei man im letzteren Fall natürlich auch das Lösungsmittel zuerst mit dem Aktivitätsstabilisator mischen und dann dem Lyophilisat zu dessen Auflösung zugeben kann.

Als immunologisch wirksame Substanz im Konjugat wird bevorzugt ein Antikörper oder ein Fragment davon, ein Antigen oder ein Hapten verwendet.

Als Antigene bzw. Haptene kommen beispielsweise in betracht Proteine, Drugs, Steroide und Hormone. Beispiele hierfür sind TBG, Cortisol, Vitamin $B_{12}$, Digoxin, Digoxigenin und Thyroxin.

Als Antikörper kommen polyklonale und monoklonale Antikörper und deren Fragmente in betracht. Ebenfalls können chemisch abgewandelte Derivate derselben, beispielsweise mit Glutardialdehyd vernetzte Antikörper Komponenten des Konjugats darstellen. Beispiele hierfür sind Antikörper gegen TSH, hCG, AFP, LH, FSH, Prolactin, Ferritin, CEA und Insulin.

Als POD kommen alle Abarten dieses Enzyms in betracht. Bevorzugt wird aufgrund ihrer guten Zugänglichkeit die Meerettich-POD.

Zweckmäßig wird beim erfindungsgemäßen Verfahren neben dem erwähnten Aktivitätsstabilisator noch ein Konservierungsmittel zugesetzt. Geeignete Konservierungsmittel sind zum Beispiel Merthiolat, Germall, Dowicil und Kathon CG.

Ein weiterer Gegenstand der Erfindung ist ein stabilisiertes Enzympräparat mit einem Gehalt an Peroxidase oder Peroxidase-Konjugat, welches dadurch gekennzeichnet ist, daß es 0,0005 bis 2 Gew.-% Aminopyrin enthält.

# EP 0 197 447 B1

Hinsichtlich der Komponenten des Konjugats, der Mengen an Aktivitätsstabilisator und eines etwaigen Gehaltes an zusätzlichem Konservierungsmittel gelten die obigen Ausführungen zum Verfahren für das stabilisierte Enzympräparat sinngemäß. Außerdem enthält das Enzympräparat zweckmäßig noch Puffersubstanzen, beispielsweise Phosphatpuffer, Citratpuffer, Boratpuffer und dergleichen oder/und Rinderserumalbumin oder/und ein System zum Nachweis der Peroxidaseaktivität.

Die Erfindung ermöglicht eine Stabilisierung der Enzymaktivität von POD oder POD-Konjugaten gegen die in Lösung normalerweise auftretende rasche Aktivitätsabnahme ohne nachteilige Beeinflussung der Immunreaktion bei den Konjugaten. Dabei gelingt erfindungsgemäß die Aktivitätsstabilisierung auch bei höheren Temperaturen über längere Zeit, so daß die Verwendbarkeit gebrauchsfertiger Lösungen von POD oder POD-Konjugaten wesentlich verbessert wird.

Die folgenden Beispiele erläutern dies näher.

Beispiel 1

Stabilisierung von Antikörper-POD-Konjugat

Lösung 1

| | |
|---|---|
| Inkubationspuffer | |
| Phosphatpuffer | 40 mmol/l, pH 7,4 |
| Rinder-Ig | 0,1 Gew.- % |
| Merthiolat | 0,01 Gew.- % |

Lösung 2

| | |
|---|---|
| Anti-Ferritin-POD-Konjugat | |
| gelöst in Lösung 1) | ca.10 U/l |

Lösung 3

| | |
|---|---|
| Substrat/Puffer-Lösung | |
| Phosphat-Citrat-Puffer | 100 mmol/1, pH 4,4 |
| Natriumperborat | 3,2 mmol/l |
| ABTS®1) | 1,9 mmol/l |

1) 2,2'-Azino-di-[3-ethyl-benzthiazolin-sulfonsäure (6)]-diammoniumsalz.

Die verwendeten Lösungen, beschichtete Röhrchen und Standard entstammen dem Enzymun-Test® Ferritin (Boehringer Mannheim GmbH, Best.-Nr. 677 337). Die Durchführung der Bestimmung erfolgt analog der Vorschrift des Herstellers.

In mit Anti-Ferritin-Antikörpern beschichteten Röhrchen werden 1 ml Lösung 1 und 0,1 ml Ferritin-Standard (ca. 440 ng/ml) gegeben und 60 Minuten bei 20 bis 25 °C inkubiert. Nach Aussaugen und Spülen wird 1 ml Lösung 2 zugegeben. Die hierzu verwendete Lösung 2 wurde entweder frisch angesetzt (Vergleichsmessung) oder vor Zusatz 24 Stunden bei 30 °C inkubiert, wobei gegebenenfalls 0,02 Gew.- % Aminopyrin zugesetzt wurde.

Nach 60 Minuten Inkubation bei 20 bis 25 °C werden die Röhrchen ausgesaugt und gespült. Anschließend wird 1 ml Lösung 3 zugegeben und wieder 60 Minuten bei 20 bis 25 °C inkubiert. Danach wird gegen Lösung 3 als Leerwert eine photometrische Bestimmung bei $\lambda$ = 405 nm durchgeführt.

Die in der nachfolgenden Tabelle 1 aufgeführten Werte sind jeweils bezogen auf den relativen Wert 100 %, der mit einem Test erhalten wurde, bei dem frisch angesetzte Lösung 2 (ohne Aminopyrin) verwendet wurde.

Analoge Messungen wurden durchgeführt mit

| | |
|---|---|
| Enzymun-Test® Ferritin | Best.-Nr 67 73 37 |
| Enzymun-Test® TBK | Best.-Nr 24 94 16 |
| Enzymun-Test® AFP | Best.-Nr 71 14 11 |
| (Hersteller : Boehringer Mannheim GmbH) | |

(Siehe Tabelle 1 Seite 4 f.)

3

# EP 0 197 447 B1

Tabelle 1

| | 0 Stunden | | 24 Stunden | |
|---|---|---|---|---|
| Aminopyrin 0,02 Gew.-% | + | – | + | – |
| POD-Konjugat | Aktivität | | | |
| Anti-Ferritin-POD | 100 % | 100 % | 90 % | 48 % |
| Anti-AFP-POD | 100 % | 100 % | 94 % | 70 % |
| $T_4$-POD (TBK-Test) | 100 % | 100 % | 94 % | 11 % |

Beispiel 2

In analoger Weise wie im Beispiel 1 werden Messungen mit dem Enzymun® -Test Ferritin bei verschiedenen Aminopyrinkonzentrationen durchgeführt. Die Ergebnisse zeigt Tabelle 2.

Tabelle 2

| Aminopyrin Konzentrationen | Aktivität nach Belastung von: | |
|---|---|---|
| | 0 Stunden 30°C | 24 Stunden 30°C |
| ohne Aminopyrin | 100 % | 50 % |
| 0,005 % | 100 % | 90 % |
| 0,02 % | 100 % | 90 % |
| 0,05 % | 100 % | 91 % |
| 0,1 % | 100 % | 86 % |
| 0,4 % | 100 % | 83 % |
| 1 % | 100 % | 101 % |

**Patentansprüche**

1. Verfahren zum Stabilisieren der Aktivität von Peroxidase in Lösung durch Zusatz eines spezifischen Aktivitätsstabilisators, dadurch gekennzeichnet, daß man dem in fester oder gelöster Form vorliegenden Enzym als Aktivitätsstabilisator Aminopyrin in einer Menge von 0,0005 bis 2 Gew.-% bezogen auf die Lösung, zusetzt.

2. Verfahren nach Anspruch 1, dadurch gekennzeichnet, daß man 0,005 bis 1 Gew.-% Stabilisator zusetzt.

3. Verfahren nach Anspruch 1 oder 2, dadurch gekennzeichnet, daß man den Stabilisator der zur Lyophilisierung bestimmten Enzymlösung zusetzt.

4

4. Verfahren nach Anspruch 1 oder 2, dadurch gekennzeichnet, daß man den Stabilisator dem mit wäßrigem Lösungsmittel rekonstituierten Lyophilisat zusetzt.

5. Verfahren nach einem der vorhergehenden Ansprüche, dadurch gekennzeichnet, daß die Peroxidase als Konjugat mit einer immunologisch wirksamen Substanz verwendet wird.

6. Verfahren nach Anspruch 5, dadurch gekennzeichnet, daß die immunologisch wirksame Substanz ein Antikörper oder ein Fragment davon, ein Antigen oder ein Hapten ist.

7. Verfahren nach Anspruch 6, dadurch gekennzeichnet, daß die immunologisch wirksame Substanz Digoxin, Digoxigenin oder Thyroxin oder ein Antikörper gegen TSH, Ferritin oder AFP ist.

8. Verfahren nach Anspruch 6, dadurch gekennzeichnet, daß die immunologisch wirksame Substanz ein Antikörper gegen AFP, Ferritin oder TSH ist.

9. Verfahren nach einem der vorhergehenden Ansprüche, dadurch gekennzeichnet, daß man zusätzlich ein Konservierungsmittel zugibt.

10. Stabilisiertes Enzympräparat mit einem Gehalt an Peroxidase, dadurch gekennzeichnet, daß es 0,0005 bis 2 Gew.- % Aminopyrin enthält.

## Claims

1. Process for the stabilisation of the activity of peroxidase in solution by addition of a specific activity stabiliser, characterised in that, to the enzyme present in solid or dissolved form, one adds, as activity stabiliser, aminopyrine in an amount of 0.0005 to 2 wt. %, referred to the solution.

2. Process according to claim 1, characterised in that one adds 0.005 to 1 wt. % of stabiliser.

3. Process according to claim 1 or 2, characterised in that one adds the stabiliser to the enzyme solution intended for the lyophilisation.

4. Process according to claim 1 or 2, characterised in that one adds the stabiliser to the lyophilisate reconstituted with aqueous solvents.

5. Process according to one of the preceding claims, characterised in that the peroxidase is used as conjugate with an immunologically effective substance.

6. Process according to claim 5, characterised in that the immunologically effective substance is an antibody or a fragment thereof, an antigen or a hapten.

7. Process according to claim 6, characterised in that the immunologically effective substance is digoxin, digoxigenin or thyroxin or an antibody against TSH, ferritin or AFP.

8. Process according to claim 6, characterised in that the immunologically effective substance is an antibody against AFP, ferritin or TSH.

9. Process according to one of the preceding claims, characterised in that one additionally adds a preserving agent.

10. Stabilised enzyme preparation with a content of peroxidase, characterised in that it contains 0.0005 to 2 wt. % of aminopyrine.

## Revendications

1. Procédé pour la stabilisation de l'activité de la peroxydase en solution par addition d'un stabilisant spécifique d'activité, caractérisé en ce que l'on ajoute à l'enzyme présente sous forme solide ou dissoute, en tant que stabilisant d'activité, de l'aminapyrine en une quantité de 0,0005 à 2 % en poids par rapport à la solution.

2. Procédé selon la revendication 1, caractérisé en ce qu'on ajoute 0,005 à 1 % en poids de stabilisant.

3. Procédé selon la revendication 1 ou 2, caractérisé en ce qu'on ajoute le stabilisant à la solution d'enzyme prévue pour la lyophilisation.

4. Procédé selon la revendication 1 ou 2, caractérisé en ce qu'on ajoute le stabilisant au lyophilisat reconstitué par un solvant aqueux.

5. Procédé selon l'une des revendications précédentes, caractérisé en ce que la peroxydase est utilisée sous forme de conjugué avec une substance à activité immunologique.

6. Procédé selon la revendication 5, caractérisé en ce que la substance à activité immunologique est un anticorps ou un fragment d'anticorps, un antigène ou un haptène.

7. Procédé selon la revendication 6, caractérisé en ce que la substance à activité immunologique est la digoxine, la digoxigénine ou la thyroxine ou un anticorps contre la TSH (hormone stimulant la thyroïde), la ferritine ou l'AFP.

8. Procédé selon la revendication 6, caractérisé en ce que la substance à activité immunologique est un anticorps contre l'AFP, la ferritine ou la TSH.

9. Procédé selon l'une des revendications précédentes, caractérisé en ce qu'on ajoute en outre un conservateur.

10. Préparation enzymatique stabilisée présentant une teneur en peroxydase, caractérisée en ce qu'elle contient 0,005 à 2 % en poids d'aminopyrine.